(19) **Europäisches Patentamt / European Patent Office / Office européen des brevets**

(11) **EP 3 244 857 B1**

(12) **EUROPEAN PATENT SPECIFICATION**

(45) Date of publication and mention of the grant of the patent:
**09.10.2019 Bulletin 2019/41**

(51) Int Cl.:
***A61F 13/511*** *(2006.01)*  ***A61F 13/51*** *(2006.01)*

(21) Application number: **15878176.5**

(22) Date of filing: **14.01.2015**

(86) International application number:
**PCT/SE2015/050025**

(87) International publication number:
**WO 2016/114693 (21.07.2016 Gazette 2016/29)**

(54) **ABSORBENT PRODUCT COMPRISING A NONWOVEN MATERIAL**

SAUGFÄHIGES PRODUKT MIT VLIESSTOFF

PRODUIT ABSORBANT COMPRENANT UN MATÉRIAU NON TISSÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.11.2017 Bulletin 2017/47**

(73) Proprietor: **Essity Hygiene and Health Aktiebolag**
**405 03 Göteborg (SE)**

(72) Inventors:
• **ABBAS, Shabira**
**S-405 03 Göteborg (SE)**
• **FERNKVIST, Maria**
**S-405 03 Göteborg (SE)**
• **NIHLSTRAND, Anna**
**S-405 03 Göteborg (SE)**

(74) Representative: **Zacco Sweden AB**
**P.O. Box 5581**
**114 85 Stockholm (SE)**

(56) References cited:
EP-A1- 2 401 993    US-A- 4 100 324
US-A- 4 704 112    US-A- 5 383 870
US-A- 5 951 535    US-A- 6 087 551
US-A1- 2010 249 741

• DATABASE WPI Derwent Publications Ltd., London, GB; AN 1997- & JP H0 931823 A (TOYO BOSEKI) 04 February 1997

**Description**

TECHNICAL FIELD

The present disclosure relates to an absorbent product comprising a nonwoven material. The nonwoven material is arranged to be in contact with skin of a user during use of the absorbent product.

BACKGROUND ART

Users of absorbent hygiene products such as diapers, sanitary products or incontinence protection garments, sometimes experience skin problems. Skin problems can be caused by forces arising from physical/mechanical interaction between the product and the user's skin. Thus, for example chafing is caused due to extra friction between the absorbent product and skin of the user.

There have been several studies to improve softness of absorbent products and especially nonwoven materials that are used on surfaces in contact with skin, such as topsheets of absorbent products. Today's absorbent products focus on providing products with a high degree of comfort by using nonwoven materials mainly providing a soft feeling towards the skin, and this is also disclosed in prior art.

For example WO2008/147264 is related to a nonwoven material for use as a body facing sheet in an absorbent article where the nonwoven material comprises at least two layers where the layer directed against the user during use of the article comprises staple fibres having a coarseness less than 1.5 dtex. This part of the sheet feels soft for the user. It is described that a soft feeling may also be achieved using fibres/filaments such as cotton, viscose, lyocell, having a high softness and textile comfort. However, these fibres/filaments bind liquid and a wet surface against the skin is left, which is not advantageous.

US 2006/0121811 relates to an absorbent product where the surface in physical contact with the skin of the user comprises fine fibres to create a soft material against the skin. Also WO2007/114742 relates to a nonwoven topsheet material comprising microfibres in contact with the skin to improve the softness against the skin.

US 5 951 535 discloses a surface material comprising a combined nonwoven fabric comprising at least two layers of a long fibre nonwoven and a short fibre nonwoven joined together. The material is used in an absorptive article, such as a disposable diaper and a sanitary napkin. The hydrophobic long fibers may have a denier of 0.5 to 6 d/f. The material may contain short fibers of different deniers and the short fibres may have a denier of 0.5 to 6 d/f. The hydrophilic short fibres may be present in an amount of 0 to 70%, preferably 0-30%.

US 4 704 112 discloses a facing material for an absorptive pad being a combined nonwoven fabric having two layers, a layer in contact with the wearers skin of 70-100 wt% hydrophobic fibres and 0-30 wt% hydrophilic fibres and a backing layer of 50-100 wt% hydrophilic fibres and 0-50 wt% hydrophobic fibres.

SUMMARY

It is an object of the present invention to provide an absorbent product comprising a nonwoven material arranged to be in contact with the skin of a user, which nonwoven material during the use of the absorbent product reduces the risk for mechanical discomfort due to friction between the nonwoven and the skin.

We have found that fine fibres with low coarseness, as described in the prior art as useful to have closest to the skin to improve the softness, are not necessarily suitable to have closest to the skin of a user of an absorbent product in presence of small amount of moisture. Fine fibres with low coarseness are soft in a dry condition, but when moisture/liquid is present fine fibres also have disadvantages.

Friction occurring between a nonwoven material and the skin of the user is different in the presence of liquid/moisture than when no liquid/moisture is present. Even a very small amount of moisture present originating from perspiration, sweat or other body fluids has an impact on the friction forces occurred between the nonwoven material and the skin of the user. It has therefore been discovered that it is really important to carefully choose the nonwoven characteristics, so that the nonwoven is able to minimize the mechanical discomfort during the overall use of the product.

It has been realized that one main reason for mechanical discomfort is related to "clinging", i.e. forces acting between the absorbent product and the human skin in the presence of moisture (perspiration, sweat, urine). Thus, it is an object of the invention to provide an absorbent product with a nonwoven material which minimizes these forces and their negative impact on the skin. The nonwoven material may be present on all parts of the absorbent product that are in contact with skin, such as for example on topsheet of the absorbent article or in case of a diaper on waist region, hip region, standing gathers, leg openings and belt. The absorbent product provides for low friction between the skin of the user and the product, both when the area is substantially dry but also when the area is moist due to perspiration and sweat or the presence of other bodily fluids. This object is achieved with the absorbent product characterized by the features of the appended claims. The object is also attained by the use of an absorbent article, characterized by the features defined in the appended claims.

The invention relates to an absorbent product comprising a nonwoven material arranged to be in contact with skin of a user during use of the absorbent product. The nonwoven material comprises at least on a skin contacting surface a mixture of non-absorbent and absorbent fibres. The absorbent fibres are present in an amount of 2-10% by weight, based on the total weight of the fibres in the nonwoven material.

The non-absorbent fibres have a coarseness from 1.8 to 10 dtex or from 2 to 7 dtex or from 4 to 7 dtex or from 5.5 to 7 dtex.

The absorbent fibres have a coarseness from 1 to 10 dtex or from 1.1 to 7 dtex or from 1.2 to 3 dtex.

When using absorbent articles, friction occurs between the skin and the surface of the absorbent article, for example a nonwoven material. The friction between the nonwoven material and skin in presence of moisture/liquid is complex and even a very small amount of moisture has a negative impact on the measured friction. The amount of moisture may be so small that the nonwoven is experienced as dry when touched. In this case moisture may be present only between the fibres and the skin, each fibre-skin interaction due to the moisture is called a wet contact.

As mentioned above, one reason for mechanical discomfort is relating to clinging, i.e. the forces acting between the absorbent product and the human skin in the presence of small amounts of moisture such as perspiration, sweat and urine. By understanding the relation between the clinging forces causing discomfort and the properties of the nonwoven materials used in absorbent products, it is possible to create a material which minimizes these forces and their negative impact on the skin. Clinging can be described as a perpendicular force acting between a solid material and a support surface in the presence of a small amount of moist. An example of clinging is a shower curtain which can easily stick to skin in presence of small amount of moisture.

To be able to reduce the friction at areas where nonwoven lies against the skin of a wearer, the nonwoven material needs to be designed so that at least the wet friction is reduced. Wet friction is experienced between a wet or moist product and skin. Wet friction can occur even at small concentrations of moist or liquid presence in the product or in the boundary between the nonwoven and the skin. Dry friction is experienced between a dry product and skin. The measurement method for determining the wet and dry friction will be described more in detail below.

By mixing non-absorbent fibres and absorbent fibres having a coarseness of between 1-10 dtex, specifically 2-7 dtex, the absorbent product exhibits a reduction in the wet friction between the absorbent product and the skin of a user. The reduction is achieved by the fact that the moist or fluid present at the interface between the nonwoven and the skin is at least partly absorbed by the absorbent fibres and thus removed from the interface and thereby avoiding clinging. When the absorbent fibres in the nonwoven absorb fluid and/or manage the moist present between the skin and the product the wet friction between the skin and nonwoven is reduced. Additionally, by having non-absorbent fibres of a coarseness of more than 1.8 dtex, the number of contacts between the nonwoven and the skin are reduced. Wet contacts are contacts between the fibres of the nonwoven and the skin, where moisture is present only at the contact points and not in the pores of the nonwoven. A material made of coarser fibres render fewer contact points with skin than a material made of finer fibres. The combination of reducing the number of wet contacts and the absorption of moisture contributes to reducing the wet friction between the skin of the user and the nonwoven.

The nonwoven material may comprise spunbond, air laid, wet laid, carded, electro spun or meltblown nonwoven, or any combination thereof. The nonwoven material may be a laminate or a combination of several types of nonwoven materials. The nonwoven material may comprise spunbond and meltblown nonwoven layers and forms a layered product spunbond - meltblown - spunbond (SMS) or spunbond - meltblown - meltblown - spunbond (SMMS).

The nonwoven material may have a basis weight from 8 to 80 g/m2, 8 to 40 g/m2 or 8 to 30 g/m2 Thus, a nonwoven material with sufficient basis weight to resist forces created by the friction is provided.

The thickness of the nonwoven material may be between 0.1 to 3 mm, 0.1 to 2.0 mm or 0.1 to 1.0 mm. In this way the nonwoven has sufficient thickness for it to be processed in an effective way.

The absorbent fibres are preferably based on cellulose including regenerated cellulose fibres such as viscose and/or lyocell fibres which are nontoxic. The non-absorbent fibres may comprise synthetic fibres, such as polyolefin-based fibres, for example fibres of polypropylene (PP) or polyethylene (PE). The synthetic fibres may be of any commercially available type and can be obtained e.g. by extrusion.

The absorbent product may be a hygienic product with skin contact such as a diaper, incontinence protection garment, sanitary napkin, panty shield. The absorbent product may also be of other type of absorbent product in which a nonwoven is arranged to be in contact with skin of the user.

The absorbent product comprises a chassis having a front and rear panel and an absorbent body having a wetting zone for receiving urine and other bodily fluids. The nonwoven material is comprised in at least one region outside the wetting zone in the absorbent body for receiving urine and other bodily fluids.

The absorbent product may comprise a waist region, hip region, standing gathers, leg openings and belt. The nonwoven material is at least comprised in at least one of the waist region, hip region, standing gathers, leg openings and belt. These areas may include moist, e.g. perspiration/sweat, and friction between the nonwoven material and the skin of the user occurs with increased risk for chafing. This risk can be decreased by the use of the nonwoven material in these regions.

The belt can be attached to the chassis or the belt can be separate from the chassis but being arranged to be attachable to the chassis. The nonwoven may at least be comprised in the belt on a side of the belt being arranged to be in contact with skin. Thus risk for chafing and skin problems can be reduced in the belt region.

The absorbent product may comprise a topsheet, an absorbent body and a backsheet, and wherein the nonwoven material is comprised in the topsheet and/or in the backsheet (for example around the leg openings in the backsheet being in contact with the skin) of the absorbent product. In this way risk for skin problems can be reduced in a large area of the absorbent product being in contact with the skin.

The nonwoven material as described above can render lower friction values in presence of moisture than a nonwoven material consisting of non-absorbent fibres having a finer coarseness than 1.8 dtex and absorbent fibres having a finer coarseness than 1 dtex. These friction values are measured according to a repeated stick and slip method which will be described in more detail below. A curve with friction value measurements is obtained in repeated runs using the method. The curve comprises a first slope having a positive coefficient illustrating increase in the friction values, a plateau, and a second slope having a negative coefficient illustrating decrease in the friction values. At the plateau, the friction values are substantially constant over the extension of the plateau. Small variations at the plateau as well as along the slopes are possible between individual values, but with a positive coefficient is meant that all individual values in the first slope together creates a positive coefficient, as well as all individual values in the second slope together creates a negative coefficient, as well as all individual values in the plateau together creates a plateau. Lower friction values render the absorbent product more skin friendly and skin problems arising with the use of the absorbent product can be reduced.

The invention further relates to the use of a nonwoven material in an absorbent or hygiene product to reduce wet friction between the nonwoven material and skin of a user. The absorbent article or hygiene product comprises a chassis having a front and rear panel and an absorbent body having a wetting zone for receiving urine and other body fluids. The nonwoven material is comprised in at least one region outside the wetting zone in the absorbent body for receiving urine and other body fluids. The nonwoven material comprises a mixture of non-absorbent and absorbent fibres, wherein the absorbent fibres are present in an amount 2-10% by weight, based on the total weight of the fibres in the nonwoven material. The non-absorbent fibres have a coarseness of 1.8 to 10 dtex and the absorbent fibres have a coarseness of from 1 to 10 dtex. It has been noted that the nonwoven material of this type with lower friction values render the absorbent product more skin friendly and skin problems arising with the use of the absorbent product can be reduced.

The wet friction is measured between the surface of the nonwoven and skin of a user of the absorbent product by the repeated stick and slip method described below.

The nonwoven material is used in an absorbent product chosen from a diaper, incontinence protection garment, sanitary napkin or panty shield.

The nonwoven material may be used substantially in non-absorbent regions of the absorbent product.

Further objects and advantages of the present invention will now be described with reference to the drawings and detailed description below.

BRIEF DESCRIPTION OF THE DRAWINGS

Fig. 1 schematically shows friction measurements made with a nonwoven with and without the mixing of absorbent fibres and non-absorbent fibres using the repeated stick and slip method;

Fig. 2 shows principle sketches of the different force contributions.

Fig. 3a and 3b show measurement data obtained by the repeated stick and slip method for a number of nonwoven materials comprising only non-absorbent fibres with varying coarsenesses and a number of nonwoven materials comprising a mixture of non-absorbent and absorbent fibres;

Fig. 3c and 3d show microscope images of two nonwovens having different coarsenesses;

Fig. 4 schematically shows an absorbent article in the form of a panty liner comprising a nonwoven material;

Fig. 5 schematically shows an absorbent article in the form of a sanitary napkin comprising a nonwoven material;

Fig. 6 schematically shows an absorbent article in the form of a diaper comprising a nonwoven material;

Fig. 7 schematically shows an absorbent article in the form of a pant-type diaper comprising a nonwoven material;

Fig. 8 schematically shows an absorbent article in the form belt-type diaper comprising a nonwoven material; and

Fig. 9 shows schematically the instrument set up for the stick and slip measurement method.

DETAILED DESCRIPTION

By "absorbent product" is meant a product that absorbs or is adapted to absorb bodily fluids, such as urine and/or blood. The absorbent product is wearable by a user and by "wearable absorbent product" is meant an absorbent article which is to be worn by the user, such as a diaper, pant-type diaper, sanitary napkin, panty-liner or incontinence product.

By "absorbent fibre" is meant a fibre having the ability to absorb liquid, such at least 1g liquid/1g fibres. The fibre also has moisture buffering capacity and is defined as hygroscopic.

By "non-absorbent fibre" is meant a fibre with substantially no absorption capacity.

The nonwoven material layers or webs of the present invention may advantageously be selected from, for example, of spunbond, air laid, wet laid, carded, electro spunned or meltblown nonwovens. The nonwoven material may be bonded by multiple techniques, e.g. by needling, hydroentangling, or heat bonding.

The nonwoven material of the disclosed products is a mixture of natural and synthetic materials. Natural fibres are for instance cellulosic fibres or fibres from regenerated cellulose. The absorbent fibres are preferably based on cellulose including regenerated cellulose fibres such as viscose and/or lyocell fibres which are nontoxic. The non-absorbent fibres may comprise synthetic fibres, such as polyolefin-based fibres, for example fibres of polypropylene (PP) or polyethylene (PE). The synthetic fibres may be of any commercially available type and can be obtained e.g. by extrusion.

The nonwoven material may be a combination of several types of nonwoven materials, such as spunbond-meltblown, spunbond - meltblown - spunbond (SMS) type or spunbond - meltblown - meltblown - spunbond (SMMS) type. In case several layers of nonwoven are laminated by means of gluing or by ultrasound, only the nonwoven layer lying against the skin is the nonwoven material referred to in the description.

The basis weight for the nonwoven material can be varied of from 8 to 80 $g/m^2$, preferably from 8 to 40 $g/m^2$, and more preferably from 8 to 30 $g/m^2$. When the basis weight is under 40 $g/m^2$, sufficient breathability, drapeability and comfort for the product can be obtained. The basis weight of from 8 to 30 $g/m^2$ has been found to provide best comfort and flexibility while processability of the material is still good.

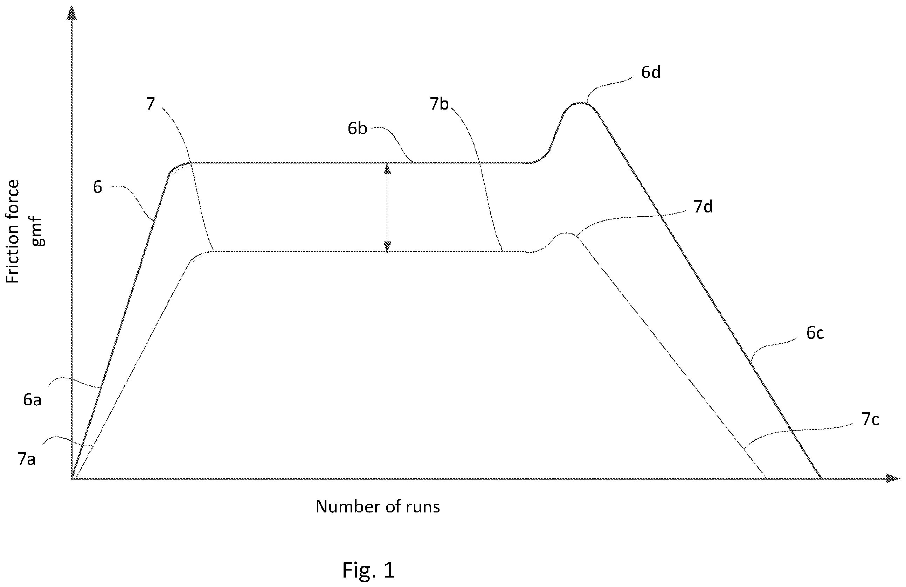

Fig. 1 schematically shows friction measurements made with a nonwoven with and without the mixing of absorbent fibres and non-absorbent fibres using the repeated stick and slip method. Fig. 1 comprises two curves: first curve 6 illustrating the friction values of a nonwoven comprising only non-absorbent fibres and second curve 7 illustrating the friction values of a nonwoven comprising a mix of non-absorbent and absorbent fibres. The friction values of curves 6, 7 are plotted in a graph with the number of friction runs on the x-axis and the friction force in gmf on the y-axis. By gmf is meant gram-force and one gram-force is 9.80665 mN.

**Stick and slip measurement method for measuring the wet friction**

The method measures the static friction, sns value (stick and slip value) in gram force, gmf, between a material and the human skin. The method means that repeatedly runs are made using the same material strip. First the sns value for the dry state (dry material and skin) is measured followed by wet state at different liquid levels (from completely wetted material, to moist and to almost dry) until the sns value is back to the skin-material interaction level measured in the first dry run, which means that the material is dry again. The method is thus called a repeated stick and slip method or sns run dry-wet-dry.

- Definition of the method

The stick and slip value is defined as the point on the force curve (gmf) where the material starts gliding over the arm. The sns values from all single force curves are then put together in a new graph, sns values as a function of number of runs.

- Principle of the method

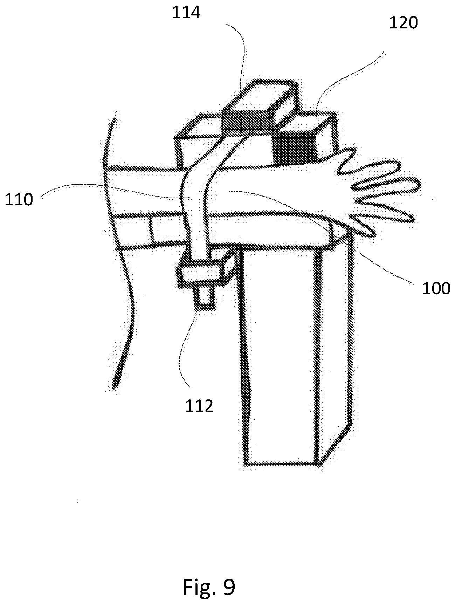

A strip 110 of test material is pulled, with the help of a MTT 170 tensile tester 120, across the volar forearm 100 to measure the static friction between the material and the skin as illustrated in Fig. 9. First a dry strip is pulled across the volar forearm. Then the strip is wetted completely and is pulled repeatedly across the volar forearm until the dry state is reached again. Systems with dry skin/dry material, dry skin/wet material, occluded skin/dry material and occluded skin/wet material can be tested. Dry skin/wet material is the only measurement made in the description, however during the wet runs the skin is to a certain degree influenced by the liquid in the system and may become somewhat occluded before finally reaching the level for the dry sns value again.

- Equipment of method

- Test person's arm, volar forearm
- The test person is acclimatized during 15 min in a climate room with 21 °C and 45%rh.
- The test is performed in a climate room with 21 °C and 45%rh
- MTT 170 tensile tester from DiaStron
- Adjustable armrest channel
- Software MTT Win (UvWin 1.32.000)
- Clamp 1: on the tensile tester
- Clamp 2: counter weight, 60 g
- 0.9 weight % NaCl solution (150 ml/material strip)
- Punch, 30*350 mm

- Material

The material to be tested is punched or made into rectangular strips measuring 30 x 350 mm. When testing, the treated side, i.e. the side of the nonwoven having claimed properties, is placed towards the skin.

- Wetting of the material strip

The material strip is completely wetted by submerging the whole strip in a beaker of 0,9 weight % NaCl solution (150 ml) for 1 min. The strip is lifted in the edge that will be placed in the clamp of the tensile tester using a pair of tweezers. The clamp of the tensile tester is illustrated in Fig. 9 with reference number 114. The strip is slowly pulled up against the edge of the beaker which allows the material to drain its excess liquid. This represents a completely wetted nonwoven, a saturation of 100%. In the other edge of the strip the counter weight of 60 g is placed. The counter weight is illustrated in Fig. 9 with reference number 112.

The sns runs with the wet strip are then tested in the same way as the first run.

- Start procedure

The computer and control unit are turned on and the instrument and program is initialized and is in the ready to start mode.

Delay time is for every friction measurement 12 seconds to give time to zero the load and place the material strip in the right position on the arm before the run begins.

If a material strip has not reached its slip value after 50 mm the distance needs to be increased.

- Positioning of test persons arm

The test person should be standing close to the instrument with the arm supported comfortably in the armrest channel. The armrest channel is adjusted so that the top of the volar forearm is in level with the clamp on the tensile tester. This means that the material strip is horizontal between the clamp and arm. During measurements, the arm should be kept still and relaxed.

- Performing the test

The dry nonwoven strip is placed in the clamp on the tensile tester and the 60 g counter weight is fastened in the other edge of the strip.

The test persons arm should be correct placed in the armrest channel as described according to "Positioning of test persons arm".

The test is started. The first seconds of the delay time is used to zero the measurement, lift the nonwoven strip from the arm and hold the counter weight so that there is no tension on the tensile tester. The nonwoven strip is then hung over the relaxed arm and the counter weight should be still. The sns run starts directly when the delay time of 12 sec. is finished._

The load cell travels the pre-set distance (50 mm) at a certain speed (150 mm/min) and pulls the nonwoven strip over the arm and when it stops the sns value is noted. The so called sns value, which is where the material no longer "sticks" to the skin and starts to glide, is noted for every repetition.

Let the tensile tester go back to the start position.

The same nonwoven strip is then submerged in a bath of 0,9 % NaCl solution for 1 min, see instructions about "Wetting of material strip". The wet nonwoven strip is then attached to the clamp on the tensile tester exactly as for the first run with the counterweight in the edge and the arm in the same position. The friction measurement is started in the same way as the first run and the sns value from the friction curve for the second run is noted.

Then the nonwoven strip is lifted away from the arm, without touching anything, while the tensile tester goes back to its start position. When back at the start position the next run will be started, in the same way as for the first run and as soon as possible, and the third sns value is noted. The test continues like this until the sns value is on the same level as the first run.

- Calculation and expression of results

The sns value from each run is noted (gmf) and a graph showing the repeated sns values (gmf) is made, sns values as a function of number of runs.

Returning to the nonwoven material, the nonwoven material with a mixture of non-absorbent and absorbent fibres and wherein the non-absorbent fibres have a coarseness of 1.8 to 10 dtex and the absorbent fibres have a coarseness of 1 to 10 dtex is represented by second curve 7 in Fig. 1 and has lower relative friction values than a nonwoven material consisting of non-absorbent fibres having a finer coarseness than 1.8 and absorbent fibres having a finer coarseness than 1 dtex represented by first curve 6. The friction values for both materials are measured according to the repeated stick and slip method on the same test person. Curves 6, 7 have friction value measurements obtained in repeated runs using the method. The curves 6, 7 comprise a first slope 6a, 7a having a positive coefficient illustrating increase in the friction values, a plateau 6b, 7b and a second slope 6c, 7c having a negative coefficient illustrating decrease in the friction values. At the plateau, the friction values are essentially the same, small variations are possible.

The curves start at a value corresponding to the dry friction measured for dry nonwoven. The positive coefficient of the first slope 6a, 7a indicates an increase in friction when the dry nonwoven is wetted and wet friction occurs. The second slope 6c, 7c illustrates the interface between the skin and nonwoven returning the friction curve to the value of dry friction. As can be seen from Fig. 1, the nonwoven material according to the invention has a lower wet friction over the entire range of runs.

For some materials a very clear peak can be seen in a curve of friction values. As indicated by references 6d and 7d in Fig. 1, these peaks are caused by clinging, which occurs when only a small amount of moisture is present. The mechanical and adhesive properties of many substances are very sensitive to the presence of moist. This is the effect of the capillarity of liquid around surface contact site, which can have a profound effect on the strength of adhesion joints and form meniscus. This is the clinging effect and clinging force. This value may be reduced with a nonwoven according to the invention.

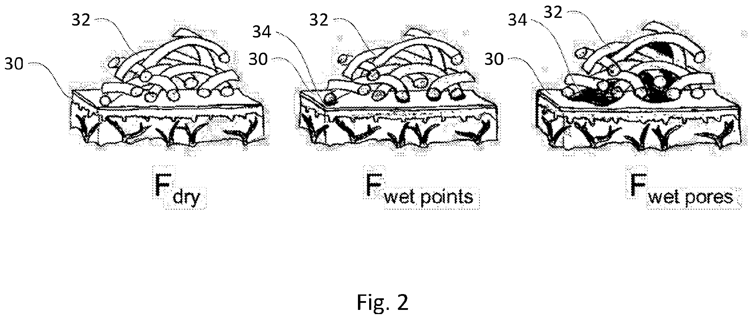

Fig. 2 shows principle sketches of the different force contributions and where on the wet-dry range they are assumed to act. From left to right: F_dry or the force exerted between dry skin 30 and a dry nonwoven material comprising fibres 32, F_wet points contacts or the force exerted between the fibres 32 of the material and the skin 30 in the presence of a small amount of moisture 34 and F_wet pores or the force exerted between a wet material comprising fibres 32 and a large amount of moisture 34 illustrated by the shadowed area and the skin 30. In the last case there is more moisture present than for when F_wet points contacts is exerted. The larger amount of moisture 34 present in the pores can be seen to create a thin film over the skin.

The total frictional force in a system involving moisture is the sum of the dry force (F_dry) and the clinging force (F_clinging):

$$F\_friction = F\_dry + F\_clinging \qquad (Eq. 1)$$

Normally F_dry << F_clinging. The clinging force can be further divided into contributions arising from wet contacts and wet pores:

$$F\_clinging = F\_wet\ contacts + F\_wet\ pores \qquad (Eq.\ 2)$$

In reality, the frictional force is a mixture of all three interactions occurring in different numbers as described by equation 3:

$$F\_friction\ (s) = F\_dry \times C\_dry + F\_wet\ contacts \times C\_wet\ contacts(s) +$$

$$+ F\_wet\ pores \times C\_wet\ pores(s) \qquad (Eq.3)$$

where s is the degree of saturation at the interface and C is the occurrence of interactions.

When the material is wetted, the force from wet pores provides the substantial contribution to the wet friction. The wet pore force rapidly increases the friction as seen by the positive coefficient of first slope 6a, 7a of curve. The material slowly dries over the next runs. Over these runs the force from the wet pores still provides the greatest contribution to the friction force. This in indicated by the plateau 6b, 7b. As the material dries further, the force from the wet contacts provides a sharp rise in the friction force, as indicated by the sharp increase of friction of peak 6d, 7d. After the peak 6d, 7d is reached, the material dries further reducing the number of wet contacts. This quickly returns the curve to the dry friction value. This is seen by the negative coefficient of second slope 6c, 7c. In some situations, there will be no peak 6d, 7d which is visible in measurements.

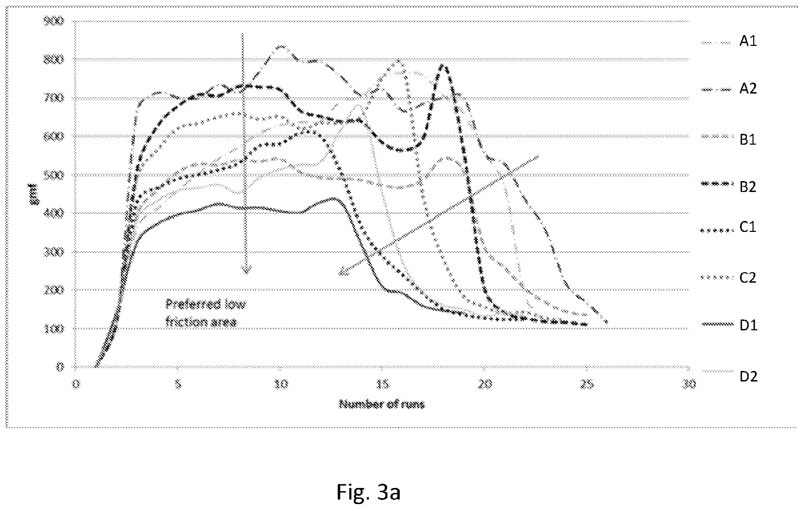

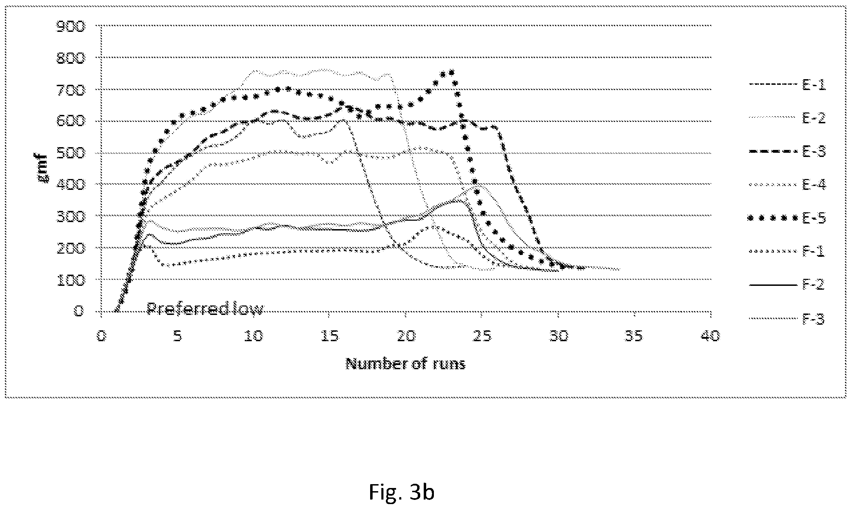

Fig. 3a and 3b show measurement data obtained by the repeated stick and slip method for a number of nonwoven materials comprising only non-absorbent fibres with varying coarseness (Fig. 3a) and a number of nonwoven materials comprising only non-absorbent fibres and a mixture of non-absorbent and absorbent fibres (Fig. 3b). Tables with measurement values for the curves of Fig. 3a and 3b can be found in Appendix 1 and 2 respectively, attached in the end of the detailed description.

|  | gsm | dtex | polymer | technology |
|---|---|---|---|---|
| **Material A** | 15 | 1,5 | PE/PP SC | Spunbond |
| **Material B** | 17 | 1,4 | PP | Spunbond |
| **Material C** | 17 | 3,6 | PP | Spunbond |
| **Material D** | 17 | 6,5 | PP | Spunbond |
| **Material E** | 18 | 3,3 | PP | Carded |
| **Material F** | 18 | 3,3 1,7 | PP 2% Viscose | Carded |

Material A, B, C, D and E are made of 100 weight percent of non-absorbent fibers and material F is made of 98 weight percent non-absorbent fibers (polypropylene) and 2 weight percent absorbent fibers (viscose fibers). PE is polyethylene and PP is polypropylene. Material A is a bicomponent fiber of polyethylene and polypropylene and SC stands for sheath/core.

Looking at Fig. 3a, overall higher friction values are obtained for low dtex material A (A1 and A2) (1.5 dtex) and material B (B1 and B2) (1.4 dtex) whereas lower friction value is obtained for a high dtex material D (D1 and D2) (6.5 dtex). This illustrates the effect of that using coarser fibres reduces the wet friction.

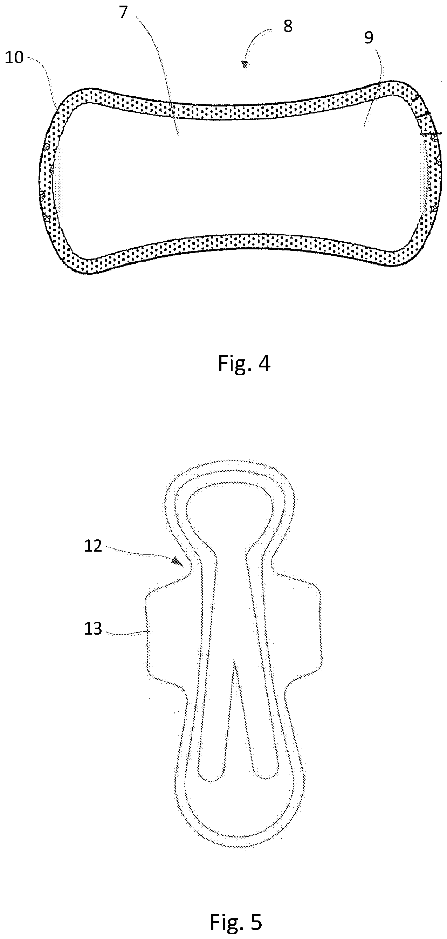

In Fig. 4b lower friction value is obtained for material F (F1,F2,F3), having 2 % of absorbent fibres (viscose), compared to material E (E1,E2,E3,E4,E5).

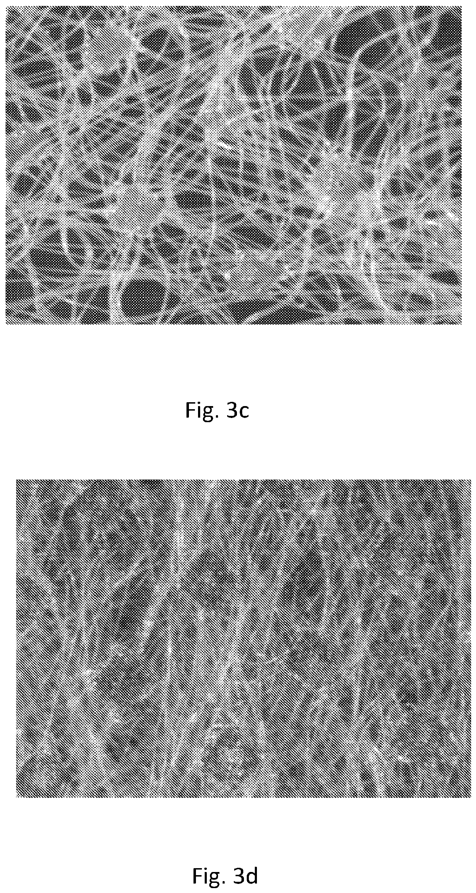

Fig. 3c and 3d show microscope images of two nonwovens having different fibre coarseness. Fig. 3c and 3d show that the nonwovens differ structurally and that their contact pattern differs due to different fibre coarseness. Fig 3c shows a microscope image of a nonwoven with a coarseness of 6.5 dtex and fig 3d shows a microscope image of a nonwoven with a coarseness of 1.4 dtex. Both nonwovens are spunbonded and made of polypropylene (PP) fibres. The surface weight is 17 gsm for both nonwovens.

As regards the carded materials in Fig. 3b, it is clear that material F (F1, F2 and F3) containing 2% viscose fibres has a considerably lower friction plateau than material E (E1,E2,E3,E4) the corresponding materials made of 100% polypropylene (PP) fibres. As described above, the viscose fibres due to their absorbency keeps the contact interface drier than the non-absorbing PP-fibres. As described in conjuncture with Fig. 1, it can be seen that the plateau level normally is followed by a sudden increase in friction (the "clinging peak") before the friction force starts to drop again. An absorbent product generally in which the nonwoven material is used comprises a chassis and an absorbent structure within the chassis. The chassis comprises a front panel and a rear panel. The front panel is intended to overlie the abdominal region of the wearer and the rear panel is intended to overlie the lower back and buttocks region. The absorbent product also has a crotch region extending between the front panel and the rear panel. The crotch region may be made of the absorbent structure and sometimes also the chassis of the product. Typically, the absorbent structure further comprises an absorbent body located primarily in the crotch region but can also extend into the front panel and the back panel of the chassis, with the absorbent body being sandwiched between a liquid pervious topsheet and a generally liquid impervious backsheet. The outer cover of the chassis may also be the liquid impervious backsheet of the absorbent structure.

The absorbent body may comprise any conventional material suitable for absorbing discharged bodily wastes, such as cellulosic fluff pulp, tissue layers, highly absorbent polymers (superabsorbents), absorbent foam materials including hydrogel-foam material, absorbent nonwoven materials or the like.

Generally, the liquid permeable topsheet comprises or consist of a nonwoven material. The topsheet material may further be composed of tow fibres, porous foams, apertured plastic films etc. As mentioned above, the materials suited as topsheet materials should be soft and non-irritating to the skin and be readily penetrated by body fluid, e.g. urine or menstrual fluid, and display low rewetting properties.

The liquid impermeable backsheet may consist of a thin plastic film, e. g. a polyethylene or polypropylene film, a nonwoven material coated with a liquid impervious material, a hydrophobic nonwoven material which resists liquid penetration or laminates of plastic films and nonwoven materials. The backsheet material may be breathable so as to allow vapour to escape from the absorbent body, while still preventing liquids from passing through the backsheet material.

The topsheet and backsheet may be connected to each other for example by adhesive bonding, gluing or welding by heat or ultrasonic. The topsheet and/or the backsheet may further be attached to the absorbent body by any method known in the art, such as adhesive, heat-bonding etc.

Below follows a number of examples of absorbent products comprising a nonwoven material. The absorbent products are wearable absorbent products. Features of the absorbent articles shared between the products have the same reference numbers. In accordance with the present invention the absorbent product, i.e. for example a conventional diaper, belted absorbent product or pant type diaper or any other absorbent product, includes at least one region comprising the nonwoven as described earlier. The region preferably at least partially comprises a waist region and/or a hip region of the absorbent product to provide comfort and fit around the waist. The hip region is defined as the region below the waist region and above the crotch region. It includes the hips, the abdominal region and the lower part of the back and the upper parts of the buttocks that are at the same height as the hips. The region may also be a leg structure in the crotch region, whereby the nonwoven can provide a reduced friction against the skin of the user when the contact surface includes moisture. The nonwoven of the present invention may also constitute at least a part of a standing gather structure in the crotch region of the absorbent product or it can be a part of a crotch elastic structure in the crotch region. The crotch elastic structure serves i.a. to encourage the absorbent product to adopt a bowl shape in the crotch region when the product is worn to thereby assist in retaining discharged bodily wastes.

The nonwoven of the present invention is especially suitable to be used at regions outside an initial wetting zone or landing zone of the absorbent products. This means that the nonwoven may at least partially cover the absorbent structure, but is located outside the initial wetting zone or landing zone, i.e. an area in the crotch portion to which urine initially lands.

Fig. 4 schematically shows an absorbent article in the form of a panty liner 8 comprising a nonwoven. By panty liner is meant an absorbent product which is used for feminine hygiene and which is narrower than sanitary napkins. Panty liners absorb less liquid than sanitary napkins and are thus aimed for light bodily discharge and for everyday cleanliness. The panty liner 8 comprises a liquid-permeable topsheet 9, a liquid-impermeable back sheet 10 and an absorbent body 11 arranged between the topsheet 9 and the back sheet 10. The absorbent body 11 comprises an absorbent material with absorbent characteristics and a superabsorbent material. The panty liner 8 has a first extension in a longitudinal direction and a second extension in a transverse direction. The panty liner 8 comprises a nonwoven having a mixture of non-absorbent and absorbent fibres, wherein the non-absorbent and/or absorbent fibres have a coarseness of 1 to 10 dtex, at least on the surface of the panty liner 8 which is in contact with the skin, below called mixed nonwoven. This surface can be the entire topsheet 9 or the part of the topsheet which lies outside the absorbent body 11 of the panty liner 8.

Fig. 5 schematically shows an absorbent article in the form of a sanitary napkin 12 comprising a mixed nonwoven as disclosed herein. The sanitary napkin 12 comprises a liquid-permeable topsheet 9, a liquid-impermeable back sheet 10 and an absorbent body 11 arranged between the topsheet 9 and the back sheet 10. The absorbent body 11 comprises an absorbent material with absorbent characteristics and a superabsorbent material. The sanitary napkin has a first extension in a longitudinal direction and a second extension in a transverse direction. The sanitary napkin may comprise wings 13, which are intended to be wrapped around the underwear of a user to secure it properly. The sanitary napkin 12 comprises a nonwoven having a mixture of non-absorbent and absorbent fibres, wherein the non-absorbent and/or absorbent fibres have a coarseness of 1 to 10 dtex, at least on the surface of the sanitary napkin 12 which is in contact with the skin. This surface can be the entire topsheet 9 or the part of the topsheet 9 which lies outside the absorbent body 11 of the sanitary napkin 12. The part of the topsheet 9 which lies outside the absorbent body 11 of the sanitary napkin 12 may comprise the wings 13.

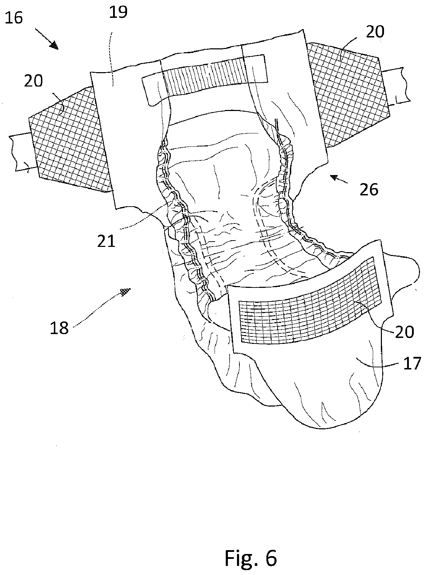

Fig. 6 schematically shows an absorbent article in the form of a diaper 16 comprising a mixed nonwoven as disclosed herein. The diaper 16 comprises a liquid-permeable topsheet 9, a liquid-impermeable back sheet 10 and an absorbent body 11 arranged between the topsheet 9 and the back sheet 10. The absorbent body 11 comprises an absorbent material with absorbent characteristics and a superabsorbent material. The diaper 16 has a first extension in a longitudinal direction and a second extension in a transverse direction. The diaper 16 comprises a chassis 26, comprising a front panel 17, a crotch region 18 and a rear panel 19. An absorbent product in the form of for example a diaper may also comprise fastening means 20 for securing the front and rear panels 17, 19 to each other to thereby secure the diaper 16 around the waist of a wearer. This type of a diaper is a conventional open diaper. The diaper 16 comprises a nonwoven having a mixture of non-absorbent and absorbent fibres, wherein the non-absorbent and/or absorbent fibres have a coarseness of 1 to 10 dtex, at least on the surface of the diaper 16 which is in contact with the skin. This surface can be the entire topsheet 9 or the part of the topsheet 9 which lies outside the absorbent body 11 of the diaper 16. The part of the topsheet 9 which lies outside the absorbent body 11 of the diaper 16 may be part of or the whole of the front panel 17, part of or whole of the rear panel 19 as well as the fastening means 20. A diaper normally comprises standing gathers 21 intended to form a leakage barrier to prevent for instance excess fluid from the absorbent body 11 to seep out from the diaper. The standing gathers 21 may also comprise the mixed nonwoven.

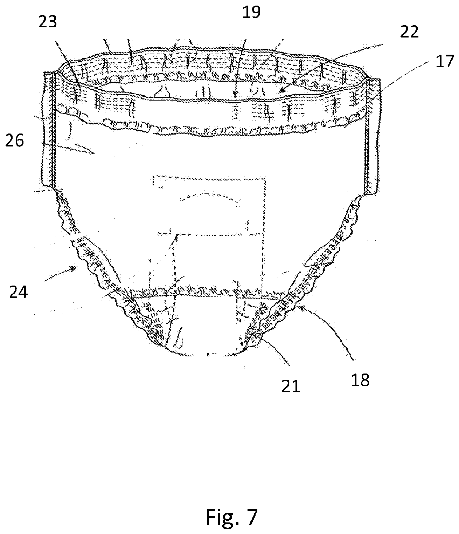

Fig. 7 schematically shows an absorbent article in the form of a pant-type diaper 22 comprising a mixed nonwowen. In contrast to a conventional open diaper, such as described in conjunction with Fig. 7, the front and rear panels 17, 19 of the chassis 26 of a pant-type diaper are initially secured to each other by means of side seams to thereby provide a garment which can be drawn up on a wearer in the same manner as a normal undergarment. The side seams may be made to be rupturable. The pant-type diaper 22 further comprises a waist elastic 23 which is arranged to secure the pant-type diaper around the waist of the wearer and leg elastic 24 which are arranged to secure the pant-type diaper around the legs of the wearer. The waist elastic 23 and leg elastic 24 may be covered by the mixed nonwoven material. In all other aspects, the pant-type diaper 22 is similar to the diaper 16, and the mixed nonwoven is placed on the same areas as described for the diaper 16.

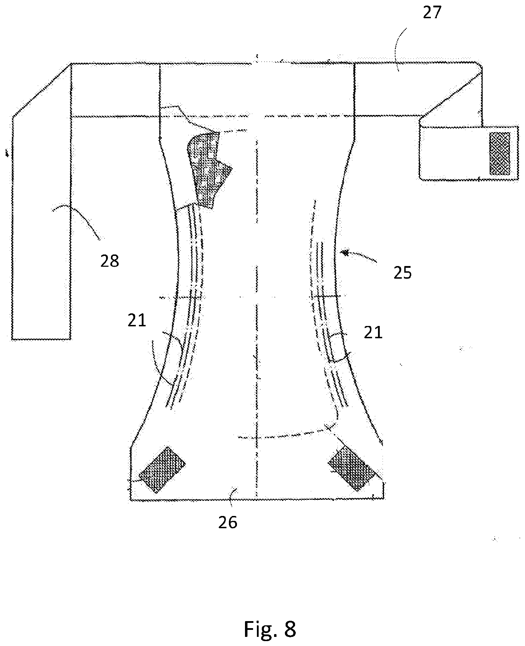

Fig. 8 schematically shows an absorbent article in the shape of a belt-diaper 25 comprising a mixed nonwoven. These products are generally worn by adults and may be adapted for both incontinence and general use. A belted absorbent product is provided with a chassis 26 and two belt halves 27, 28. The chassis of belt-diaper 25 comprises a front panel 17, a crotch region 18 and a rear panel 19. The chassis 26 may also comprise standing gathers 21. The belt halves 27, 28 are either attached to the chassis 26 extending from the lateral sides of the rear panel 19 or are separate from the chassis 26 and arranged to be attached to the chassis 26. The belt halves 27, 28 are intended to be placed around the waist of a wearer and fastened using any suitable fastener to retain the belted absorbent product around the waist of a wearer. Besides the areas of the chassis which may be covered by the mixed nonwoven, the belt on a side of the belt being arranged to be in contact with skin may also comprise the mixed nonwoven.

The above description defines examples of embodiments of the present invention but the examples are not to be regarded as limiting the invention in any way. The invention may be varied within the scope of the appended claims.

Appendix 1. Test results for nonwoven with non-absorbent fibres having different coarseness

| Sample | A1 | A2 | B1 | B2 | C1 | C2 | D1 | D2 |
|---|---|---|---|---|---|---|---|---|
| No. of runs | gmf | gmf | gmf | gmf | gmf | gmf | gmf | gmf |
| 1 | 118 | 113 | 133 | 113 | 126 | 125 | 142 | 133 |
| 2 | 362 | 667 | 396 | 513 | 430 | 493 | 324 | 382 |
| 3 | 412 | 714 | 460 | 627 | 466 | 565 | 373 | 433 |
| 4 | 459 | 702 | 508 | 679 | 490 | 621 | 396 | 459 |
| 5 | 503 | 700 | 529 | 709 | 501 | 634 | 408 | 466 |
| 6 | 543 | 735 | 526 | 706 | 513 | 651 | 424 | 475 |
| 7 | 573 | 715 | 539 | 731 | 531 | 659 | 413 | 453 |
| 8 | 608 | 769 | 535 | 729 | 576 | 644 | 414 | 493 |
| 9 | 629 | 834 | 542 | 722 | 581 | 652 | 406 | 515 |

(continued)

| Sample | A1 | A2 | B1 | B2 | C1 | C2 | D1 | D2 |
|---|---|---|---|---|---|---|---|---|
| No. of runs | gmf | gmf | gmf | gmf | gmf | gmf | gmf | gmf |
| 10 | 637 | 795 | 507 | 667 | 609 | 620 | 402 | 527 |
| 11 | 640 | 794 | 493 | 652 | 600 | 635 | 429 | 534 |
| 12 | 693 | 756 | 491 | 639 | 506 | 635 | 428 | 618 |
| 13 | 704 | 706 | 486 | 639 | 364 | 653 | 318 | 675 |
| 14 | 755 | 724 | 473 | 584 | 289 | 751 | 210 | 456 |
| 15 | 766 | 667 | 468 | 565 | 240 | 786 | 192 | 272 |
| 16 | 760 | 685 | 488 | 594 | 191 | 444 | 159 | 195 |
| 17 | 706 | 701 | 544 | 786 | 151 | 280 | 147 | 161 |
| 18 | 661 | 700 | 509 | 560 | 136 | 184 | 141 | 150 |
| 19 | 557 | 557 | 313 | 205 | 127 | 157 |  | 133 |
| 20 | 471 | 529 | 258 | 142 | 124 | 140 |  | 140 |
| 21 | 186 | 436 | 204 | 127 | 124 | 143 |  | 134 |
| 22 | 119 | 355 | 168 | 118 |  | 127 |  |  |
| 23 |  | 216 | 144 | 116 |  | 123 |  |  |
| 24 |  | 169 | 136 | 111 |  |  |  |  |
| 25 |  | 116 |  |  |  |  |  |  |

Appendix 2. Test results for nonwoven having only non-absorbent fibres and nonwoven with a mixture of non-absorbent fibres and absorbent fibres

| Sample | E1 | E2 | E3 | E4 | E5 | F1 | F2 | F3 |
|---|---|---|---|---|---|---|---|---|
| No. of runs | gmf | gmf | gmf | gmf | gmf | gmf | gmf | gmf |
| 1 | 135 | 154 | 134 | 126 | 134 | 151 | 132 | 139 |
| 2 | 353 | 434 | 383 | 309 | 445 | 208 | 239 | 279 |
| 3 | 409 | 527 | 446 | 353 | 543 | 148 | 217 | 264 |
| 4 | 464 | 579 | 472 | 383 | 610 | 150 | 214 | 253 |
| 5 | 495 | 617 | 505 | 417 | 627 | 157 | 227 | 260 |
| 6 | 520 | 628 | 548 | 461 | 643 | 163 | 232 | 261 |
| 7 | 527 | 673 | 567 | 463 | 669 | 168 | 244 | 261 |
| 8 | 556 | 706 | 594 | 473 | 675 | 176 | 244 | 256 |
| 9 | 603 | 756 | 600 | 485 | 677 | 182 | 263 | 263 |
| 10 | 591 | 744 | 630 | 503 | 692 | 184 | 259 | 278 |
| 11 | 602 | 756 | 627 | 505 | 705 | 187 | 272 | 269 |
| 12 | 552 | 745 | 610 | 498 | 690 | 191 | 261 | 263 |
| 13 | 558 | 758 | 609 | 499 | 684 | 190 | 260 | 272 |
| 14 | 569 | 759 | 621 | 468 | 673 | 192 | 259 | 277 |
| 15 | 602 | 744 | 646 | 505 | 651 | 194 | 258 | 271 |

(continued)

| Sample | E1 | E2 | E3 | E4 | E5 | F1 | F2 | F3 |
|---|---|---|---|---|---|---|---|---|
| No. of runs | gmf | gmf | gmf | gmf | gmf | gmf | gmf | gmf |
| 16 | 482 | 753 | 634 | 495 | 614 | 191 | 255 | 279 |
| 17 | 342 | 732 | 606 | 487 | 645 | 189 | 261 | 273 |
| 18 | 238 | 743 | 609 | 487 | 647 | 208 | 280 | 276 |
| 19 | 186 | 557 | 592 | 506 | 647 | 214 | 289 | 295 |
| 20 | 156 | 383 | 593 | 516 | 671 | 254 | 292 | 305 |
| 21 | 140 | 263 | 574 | 506 | 724 | 267 | 326 | 333 |
| 22 | 139 | 165 | 590 | 484 | 753 | 244 | 345 | 350 |
| 23 | 144 | 144 | 602 | 355 | 526 | 224 | 336 | 380 |
| 24 | | 132 | 576 | 250 | 320 | 177 | 212 | 394 |
| 25 | | 137 | 574 | 202 | 244 | 148 | 165 | 341 |
| 26 | | | 416 | 154 | 200 | 144 | 142 | 258 |
| 27 | | | 310 | 139 | 176 | | 135 | 207 |
| 28 | | | 185 | 133 | 156 | | 130 | 186 |
| 29 | | | 152 | | 144 | | 128 | 156 |
| 30 | | | 139 | | 133 | | | 144 |
| 31 | | | 136 | | | | | 140 |
| 32 | | | | | | | | 137 |
| 33 | | | | | | | | 132 |

**Claims**

1. Absorbent product (8; 12, 16; 22; 25) comprising a chassis (26) having a front and rear panel (17, 19) and an absorbent body (11) having a wetting zone for receiving urine and other bodily fluids, the absorbent product comprising a nonwoven material (1) arranged to be in contact with skin of a user during use of the absorbent product (8; 12, 16; 22; 25), wherein the nonwoven material (1) is located outside the wetting zone and comprises at least on a skin contacting surface a mixture of non-absorbent and absorbent fibres, wherein the absorbent fibres are present in an amount of 2-10% by weight, based on the total weight of the fibres in the nonwoven material (1), and wherein the non-absorbent fibres have a coarseness of 3.5 to 10 dtex and the absorbent fibres have a coarseness of 1 to 10 dtex.

2. Absorbent product (8; 12, 16; 22; 25) comprising a chassis (26) having a front and rear panel (17, 19) and an absorbent body (11) having a wetting zone for receiving urine and other bodily fluids, the absorbent product comprising a nonwoven material (1) arranged to be in contact with skin of a user during use of the absorbent product (8; 12, 16; 22; 25), wherein the nonwoven material (1) is located only outside the wetting zone and comprises at least on a skin contacting surface a mixture of non-absorbent and absorbent fibres, wherein the absorbent fibres are present in an amount of 2-10% by weight, based on the total weight of the fibres in the nonwoven material (1), and wherein the non-absorbent fibres have a coarseness of 1.8 to 10 dtex and the absorbent fibres have a coarseness of 1 to 10 dtex.

3. Absorbent product (8; 12, 16; 22; 25) according to claim 1, wherein the non-absorbent fibres have a coarseness of 3.5 to 7 dtex, or 5.5 to 7 dtex.

4. Absorbent product (8; 12, 16; 22; 25) according to claim 2, wherein the non-absorbent fibres have a coarseness of 2 to 7 dtex or 3.5 to 7 dtex, or 5.5 to 7 dtex.

5.  Absorbent product (8; 12, 16; 22; 25) according to any of claims 1 to 4, wherein the absorbent fibres have a coarseness of 1.1 to 7 dtex or 1.2 to 3 dtex.

6.  Absorbent product (8; 12, 16; 22; 25) according to any one of the preceding claims, wherein the absorbent fibres comprise cellulosic fibres.

7.  Absorbent product (8; 12, 16; 22; 25) according to any one of the preceding claims, wherein the nonwoven material (1) at least is comprised in a region outside the absorbent body (11).

8.  Absorbent product (16; 22; 25) according to any one of the preceding claims, wherein the absorbent product (16; 22; 25) comprises a waist region, hip region, standing gathers, leg openings and belt, and the nonwoven material (1) is comprised in at least one of the waist region, hip region, standing gathers, leg openings and belt.

9.  Absorbent product (25) according to claim 8, wherein the belt is attached to the chassis or the belt is separate from the chassis arranged to be attachable to the chassis, wherein the nonwoven is comprised in the belt on a side of the belt being arranged to be in contact with skin.

10. Absorbent product (8; 12; 16; 22; 25) according to any one of the preceding claims, wherein the absorbent product (8; 12; 16; 22; 25) comprises a topsheet (9), an absorbent body (11) and a backsheet (10), and wherein the nonwoven material (1) is comprised in the topsheet (9) and/or the backsheet (10) of the absorbent product (8; 12; 16; 22; 25).

11. Absorbent product (8; 12; 16; 22; 25) according to any one of the preceding claims, wherein the nonwoven material (1) has lower friction values in the presence of moisture, than a nonwoven material consisting of non-absorbent fibres having a finer coarseness than 1.8 dtex and absorbent fibres having a finer coarseness than 1 dtex , measured by means of a curve obtained in repeated runs with measurements according to a repeated stick and slip method described in the description, wherein the friction values in the curve are obtained in repeated runs and wherein the curve comprises a first slope having a positive coefficient illustrating increase in the friction values, a plateau, and a second slope having a negative coefficient illustrating decrease in the friction values.

12. Absorbent product (8; 12, 16; 22; 25) comprising a chassis (26) having a front and rear panel (17, 19) and an absorbent body (11) having a wetting zone for receiving urine and other bodily fluids, the absorbent product comprising a carded nonwoven material (1) arranged to be in contact with skin of a user during use of the absorbent product (8; 12, 16; 22; 25), wherein the nonwoven material (1) has a basis weight of 18 g/m$^2$, is located outside the wetting zone and comprises at least on a skin contacting surface a mixture of non-absorbent fibers comprising polypropylene and absorbent fibres comprising viscose, wherein the absorbent fibres are present in an amount of 2% by weight, based on the total weight of the fibres in the nonwoven material (1), and wherein the non-absorbent fibres have a coarseness of 3.3 dtex and the absorbent fibres have a coarseness of 1.7 dtex.

13. Use of a nonwoven material (1) in an absorbent or hygiene product comprising a chassis (26) having a front and rear panel (17, 19) and an absorbent body (11) having a wetting zone for receiving urine and other bodily fluids to reduce wet friction between the nonwoven material (1) and skin of a user, the nonwoven material (1) being at least being comprised in a region outside the wetting zone and comprising a mixture of non-absorbent and absorbent fibres, wherein the absorbent fibres are present in an amount of 2-10% by weight, based on the total weight of the fibres in the nonwoven material (1), and wherein the non-absorbent fibres having a coarseness of 3.5 to 10 dtex and the absorbent fibres have a coarseness of from 1 to 10 dtex.

14. Use according to claim 13, wherein the nonwoven material (1) is used in an absorbent product (8; 12; 16; 22; 25) chosen from a diaper, incontinence protection garment, sanitary napkin or panty shield.

15. Use according to claim 14, wherein the nonwoven material (1) is used in substantially non-absorbent regions of the absorbent product (8; 12; 16; 22; 25).

**Patentansprüche**

1.  Absorbierendes Produkt (8; 12; 16; 22; 25), das ein Chassis (26) mit einer Vorder- und Rückwand (17, 19) und einen Absorptionskörper (11) mit einer Benetzungszone zur Aufnahme von Urin und anderen Körperflüssigkeiten umfasst, wobei das absorbierende Produkt ein Vliesmaterial (1) umfasst, das so angeordnet ist, dass es mit der Haut eines Verwenders während der Verwendung des absorbierenden Produkts (8; 12; 16; 22; 25) in Kontakt steht, wobei das Vliesmaterial (1) außerhalb der Benetzungszone angeordnet ist und mindestens an einer Oberfläche, die mit der Haut in Kontakt kommt, eine Mischung aus nicht-absorbierenden und absorbierenden Fasern umfasst, wobei die absorbierenden Fasern in einer Menge von 2-10 Gewichts-% vorhanden sind, bezogen auf das Gesamtgewicht der Fasern in dem Vliesmaterial (1), und wobei die nicht-absorbierenden Fasern eine Rauheit von 3,5 bis 10 dtex aufweisen und die absorbierenden Fasern eine Rauheit von 1 bis 10 dtex aufweisen.

2. Absorbierendes Produkt (8; 12; 16; 22; 25), das ein Chassis (26) mit einer Vorder- und Rückwand (17, 19) und einen Absorptionskörper (11) mit einer Benetzungszone zur Aufnahme von Urin und anderen Körperflüssigkeiten umfasst, wobei das absorbierende Produkt ein Vliesmaterial (1) umfasst, das so angeordnet ist, dass es mit der Haut eines Verwenders während der Verwendung des absorbierenden Produkts (8; 12; 16; 22; 25) in Kontakt steht, wobei das Vliesmaterial (1) nur außerhalb der Benetzungszone angeordnet ist und mindestens an einer Oberfläche, die mit der Haut in Kontakt kommt, eine Mischung aus nicht-absorbierenden und absorbierenden Fasern umfasst, wobei die absorbierenden Fasern in einer Menge von 2-10 Gewichts-% vorhanden sind, bezogen auf das Gesamtgewicht der Fasern in dem Vliesmaterial (1), und wobei die nicht-absorbierenden Fasern eine Rauheit von 1,8 bis 10 dtex aufweisen und die absorbierenden Fasern eine Rauheit von 1 bis 10 dtex aufweisen.

3. Absorbierendes Produkt (8; 12; 16; 22; 25) gemäß Anspruch 1, wobei die nicht-absorbierenden Fasern eine Rauheit von 3,5 bis 7 dtex oder 5,5 bis 7 dtex aufweisen.

4. Absorbierendes Produkt (8; 12; 16; 22; 25) gemäß Anspruch 2, wobei die nicht-absorbierenden Fasern eine Rauheit von 2 bis 7 dtex oder 3,5 bis 7 dtex oder 5,5 bis 7 dtex aufweisen.

5. Absorbierendes Produkt (8; 12; 16; 22; 25) gemäß mindestens einem der Ansprüche 1 bis 4, wobei die absorbierenden Fasern eine Rauheit von 1,1 bis 7 dtex oder 1,2 bis 3 dtex aufweisen.

6. Absorbierendes Produkt (8; 12; 16; 22; 25) gemäß mindestens einem der vorhergehenden Ansprüche, wobei die absorbierenden Fasern Cellulosefasern umfassen.

7. Absorbierendes Produkt (8; 12; 16; 22; 25) gemäß mindestens einem der vorhergehenden Ansprüche, wobei das Vliesmaterial (1) mindestens in einem Bereich außerhalb des Absorptionskörpers (11) umfasst ist.

8. Absorbierendes Produkt (16; 22; 25) gemäß mindestens einem der vorhergehenden Ansprüche, wobei das absorbierende Produkt (16; 22; 25) einen Taillenbereich, einen Hüftbereich, stehende Falten, Beinöffnungen und einen Gürtel umfasst, und das Vliesmaterial (1) in mindestens einem Bereich aus dem Taillenbereich, dem Hüftbereich, den stehenden Falten, den Beinöffnungen und dem Gürtel umfasst ist.

9. Absorbierendes Produkt (25) gemäß Anspruch 8, wobei der Gürtel an dem Chassis befestigt ist oder der Gürtel getrennt von dem Chassis so angeordnet ist, dass er an dem Chassis befestigt werden kann, wobei das Vlies in dem Gürtel umfasst ist auf einer Seite des Gürtels, die mit der Haut in Kontakt steht.

10. Absorbierendes Produkt (8; 12; 16; 22; 25) gemäß mindestens einem der vorhergehenden Ansprüche, wobei das absorbierende Produkt (8; 12; 16; 22; 25) eine obere Lage (9), einen Absorptionskörper (11) und eine untere Lage (10) umfasst, und wobei das Vliesmaterial (1) in der oberen Lage (9) und/oder der unteren Lage (10) des absorbierenden Produkts (8; 12; 16; 22; 25) umfasst ist.

11. Absorbierendes Produkt (8; 12; 16; 22; 25) gemäß mindestens einem der vorhergehenden Ansprüche, wobei das Vliesmaterial (1) niedrigere Reibungswerte in der Gegenwart von Feuchtigkeit aufweist als ein Vliesmaterial, das aus nicht-absorbierenden Fasern mit einer geringeren Rauheit als 1,8 dtex und absorbierenden Fasern mit einer geringeren Rauheit als 1 dtex besteht, gemessen durch eine Kurve, die in Wiederholungsläufen mit Messungen gemäß eines wiederholten Haft- und Gleitverfahrens (stick and slip method) wie in der Beschreibung beschrieben erhalten wurde, wobei die Reibungswerte in der Kurve in Wiederholungsläufen erhalten wurden und wobei die Kurve eine erste Steigung mit einem positiven Koeffizienten, der den Anstieg der Reibungswerte angibt, ein Plateau und eine zweite Steigung mit einem negativen Koeffizienten, der den Abfall der Reibungswerte angibt, umfasst.

12. Absorbierendes Produkt (8; 12; 16; 22; 25), das ein Chassis (26) mit einer Vorder- und Rückwand (17, 19) und einen Absorptionskörper (11) mit einer Benetzungszone zur Aufnahme von Urin und anderen Körperflüssigkeiten umfasst, wobei das absorbierende Produkt ein kardiertes Vliesmaterial (1) umfasst, das so angeordnet ist, dass es mit der Haut eines Verwenders während der Verwendung des absorbierenden Produkts (8; 12; 16; 22; 25) in Kontakt steht, wobei das Vliesmaterial (1) ein Grundgewicht von 18 g/m$^2$ aufweist, außerhalb der Benetzungszone angeordnet ist und mindestens an einer Oberfläche, die mit der Haut in Kontakt kommt, eine Mischung aus nicht-absorbierenden Fasern, die Polypropylen umfassen, und absorbierenden Fasern, die Viskose umfassen, umfasst, wobei die absorbierenden Fasern in einer Menge von 2 Gewichts-% vorhanden sind, bezogen auf das Gesamtgewicht der Fasern in dem Vliesmaterial (1), und wobei die nicht-absorbierenden Fasern eine Rauheit von 3,3 dtex aufweisen und die absorbierenden Fasern eine Rauheit von 1,7 dtex aufweisen.

13. Verwendung eines Vliesmaterials (1) in einem absorbierenden Produkt oder Hygieneprodukt, das ein Chassis (26) mit einer Vorder- und Rückwand (17, 19) und einen Absorptionskörper (11) mit einer Benetzungszone zur Aufnahme von Urin und anderen Körperflüssigkeiten umfasst, zur Verringerung von Nassreibung zwischen dem Vliesmaterial (1) und der Haut eines Verwenders, wobei das Vliesmaterial (1) mindestens in einem Bereich außerhalb der Benetzungszone umfasst ist und eine Mischung aus nicht-absorbierenden und absorbierenden Fasern umfasst, wobei die absorbierenden Fasern in einer Menge von 2-10 Gewichts-% vorhanden sind, bezogen auf das Gesamtgewicht der Fasern in dem Vliesmaterial (1), und wobei die nicht-absorbierenden Fasern eine Rauheit von 3,5 bis 10 dtex aufweisen und die absorbierenden Fasern eine Rauheit von 1 bis 10 dtex aufweisen.

14. Verwendung gemäß Anspruch 13, wobei das Vliesmaterial (1) in einem absorbierenden Produkt (8; 12; 16; 22; 25), ausgewählt aus einer Windel, Inkontinenzschutzbekleidung, Binde oder Slipeinlage, verwendet wird.

15. Verwendung gemäß Anspruch 14, wobei das Vliesmaterial (1) im Wesentlichen in nicht-absorbierenden Bereichen des absorbierenden Produkts (8; 12; 16; 22; 25) verwendet wird.

## Revendications

1. Produit absorbant (8 ; 12, 16 ; 22 ; 25) comprenant un châssis (26) ayant un panneau avant et un panneau arrière (17, 19) et un corps absorbant (11) ayant une zone de mouillage pour recevoir de l'urine et d'autres fluides corporels, le produit absorbant comprenant un matériau non tissé (1) conçu pour être en contact avec la peau d'un utilisateur durant l'utilisation du produit absorbant (8 ; 12, 16 ; 22 ; 25), dans lequel le matériau non tissé (1) est situé à l'extérieur de la zone de mouillage et comprend, au moins sur une surface en contact avec la peau, un mélange de fibres non absorbantes et absorbantes, dans lequel les fibres absorbantes sont présentes en une quantité comprise entre 2 et 10 % en poids, sur la base du poids total des fibres dans le matériau non tissé (1) et dans lequel les fibres non absorbantes présentent une grosseur de grain comprise entre 3,5 et 10 dtex et les fibres absorbantes présentent une grosseur de grain comprise entre 1 et 10 dtex.

2. Produit absorbant (8 ; 12, 16 ; 22 ; 25) comprenant un châssis (26) ayant un panneau avant et un panneau arrière (17, 19) et un corps absorbant (11) ayant une zone de mouillage pour recevoir de l'urine et d'autres fluides corporels, le produit absorbant comprenant un matériau non tissé (1) conçu pour être en contact avec la peau d'un utilisateur durant l'utilisation du produit absorbant (8 ; 12, 16 ; 22 ; 25), dans lequel le matériau non tissé (1) est situé seulement à l'extérieur de la zone de mouillage et comprend, au moins sur une surface en contact avec la peau, un mélange de fibres non absorbantes et absorbantes, dans lequel les fibres absorbantes sont présentes en une quantité comprise entre 2 et 10 % en poids, sur la base du poids total des fibres dans le matériau non tissé (1) et dans lequel les fibres non absorbantes présentent une grosseur de grain comprise entre 1,8 et 10 dtex et les fibres absorbantes présentent une grosseur de grain comprise entre 1 et 10 dtex.

3. Produit absorbant (8 ; 12, 16 ; 22 ; 25) selon la revendication 1, dans lequel les fibres non absorbantes présentent une grosseur de grain comprise entre 3,5 et 7 dtex ou entre 5,5 et 7 dtex.

4. Produit absorbant (8 ; 12, 16 ; 22 ; 25) selon la revendication 2, dans lequel les fibres non absorbantes présentent une grosseur de grain comprise entre 2 et 7 dtex, entre 3,5 et 7 dtex, ou entre 5,5 et 7 dtex.

5. Produit absorbant (8 ; 12, 16 ; 22 ; 25) selon l'une quelconque des revendications 1 à 4, dans lequel les fibres absorbantes présentent une grosseur de grain comprise entre 1,1 et 7 dtex ou entre 1,2 et 3 dtex.

6. Produit absorbant (8 ; 12, 16 ; 22 ; 25) selon l'une quelconque des revendications précédentes, dans lequel les fibres absorbantes comprennent des fibres cellulosiques.

**7.** Produit absorbant (8 ; 12, 16 ; 22 ; 25) selon l'une quelconque des revendications précédentes, dans lequel le matériau non tissé (1) est au moins inclus dans une région à l'extérieur du corps absorbant (11) .

**8.** Produit absorbant (16 ; 22 ; 25) selon l'une quelconque des revendications précédentes, dans lequel le produit absorbant (16 ; 22 ; 25) comprend une région de taille, une région de hanche, des plis relevés, des ouvertures pour les jambes et une ceinture et le matériau non tissé (1) est inclus dans la région de taille et/ou la région de hanche et/ou les plis relevés et/ou les ouvertures pour les jambes et/ou à la ceinture.

**9.** Produit absorbant (25) selon la revendication 8, dans lequel la ceinture est fixée au châssis ou la ceinture est séparée du châssis conçue pour pouvoir être fixée au châssis, dans lequel le tissu non tissé est inclus dans la ceinture sur un côté de la ceinture qui est conçue pour être en contact avec la peau.

**10.** Produit absorbant (8 ; 12 ; 16 ; 22 ; 25) selon l'une quelconque des revendications précédentes, le produit absorbant (8 ; 12 ; 16 ; 22 ; 25) comprenant une feuille supérieure (9), un corps absorbant (11) et une feuille de support (10) et le matériau non tissé (1) étant inclus dans la feuille supérieure (9) et/ou la feuille de support (10) du produit absorbant (8 ; 12 ; 16 ; 22 ; 25).

**11.** Produit absorbant (8 ; 12 ; 16 ; 22 ; 25) selon l'une quelconque des revendications précédentes, dans lequel le matériau non tissé (1) présente des valeurs de friction plus faibles en présence d'humidité qu'un matériau non tissé se composant de fibres non absorbantes ayant une grosseur de grain plus fine que 1,8 dtex et des fibres absorbantes ayant une grosseur de grain plus fine que 1 dtex, mesurées au moyen d'une courbe obtenue lors de courses répétées avec des mesures en fonction d'un procédé de glissement saccadé répété décrit dans la description, dans lequel les valeurs de friction dans la courbe sont obtenues lors de courses répétées et dans lequel la courbe comprend une première pente ayant un coefficient positif illustrant une augmentation des valeurs de friction, un plateau et une seconde pente ayant un coefficient négatif illustrant une baisse des valeurs de friction.

**12.** Produit absorbant (8 ; 12, 16 ; 22 ; 25) comprenant un châssis (26) ayant un panneau avant et un panneau arrière (17, 19) et un corps absorbant (11) ayant une zone de mouillage pour recevoir de l'urine et d'autres fluides corporels, le produit absorbant comprenant un matériau non tissé cardé (1) conçu pour être en contact avec la peau d'un utilisateur durant l'utilisation du produit absorbant (8 ; 12, 16 ; 22 ; 25), dans lequel le matériau non tissé (1) présente un poids de base de 18 g/m$^2$, est situé à l'extérieur de la zone de mouillage et comprend, au moins sur une surface en contact avec la peau, un mélange de fibres non absorbantes comprenant du polypropylène et de fibres absorbantes comprenant de la viscose, dans lequel les fibres absorbantes sont présentes en une quantité de 2 % en poids, sur la base du poids total des fibres dans le matériau non tissé (1) et dans lequel les fibres non absorbantes présentent une grosseur de grain de 3,3 dtex et les fibres absorbantes présentent une grosseur de grain de 1,7 dtex.

**13.** Utilisation d'un matériau non tissé (1) dans un produit absorbant ou hygiénique comprenant un châssis (26) ayant un panneau avant et un panneau arrière (17, 19) et un corps absorbant (11) ayant une zone de mouillage pour recevoir de l'urine et d'autres fluides corporels pour réduire la friction humide entre le matériau non tissé (1) et la peau d'un utilisateur, le matériau non tissé (1) étant au moins étant inclus dans une région à l'extérieur de la zone de mouillage et comprenant un mélange de fibres non absorbantes et absorbantes, dans lequel les fibres absorbantes sont présentes en une quantité comprise entre 2 et 10 % en poids, sur la base du poids total des fibres dans le matériau non tissé (1) et dans lequel les fibres non absorbantes présentent une grosseur de grain comprise entre 3,5 et 10 dtex et les fibres absorbantes présentent une grosseur de grain comprise entre 1 et 10 dtex.

**14.** Utilisation selon la revendication 13, dans laquelle le matériau non tissé (1) est utilisé dans un produit absorbant (8 ; 12 ; 16 ; 22 ; 25) choisi parmi une couche, un vêtement de protection contre l'incontinence, une serviette hygiénique ou un protège-culotte.

**15.** Utilisation selon la revendication 14, dans laquelle le matériau non tissé (1) est utilisé dans des régions sensiblement non absorbantes du produit absorbant (8 ; 12 ; 16 ; 22 ; 25).

Fig. 1

EP 3 244 857 B1

$F_{dry}$     $F_{wet\ points}$     $F_{wet\ pores}$

Fig. 2

EP 3 244 857 B1

Fig. 3a

Fig. 3c

Fig. 3d

Fig. 4

Fig. 5

Fig. 6

Fig. 7

27

28

25

21

21

26

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008147264 A
- US 20060121811 A
- WO 2007114742 A
- US 5951535 A
- US 4704112 A